(19) Europäisches Patentamt
European Patent Office
Office européen des brevets

(11) **EP 2 249 181 A1**

(12) **EUROPEAN PATENT APPLICATION**
published in accordance with Art. 153(4) EPC

(43) Date of publication:
**10.11.2010  Bulletin 2010/45**

(51) Int Cl.:
***G01T 1/161*** (2006.01)

(21) Application number: **09710106.7**

(22) Date of filing: **15.01.2009**

(86) International application number:
**PCT/JP2009/000121**

(87) International publication number:
**WO 2009/101759 (20.08.2009 Gazette 2009/34)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority:  **13.02.2008   JP 2008031922**

(71) Applicant: **Nihon Medi-Physics Co., Ltd.**
**Tokyo 136-0075 (JP)**

(72) Inventors:
• **ABURANO, Tamio**
**Asahikawa-shi**
**Hokkaido 078-8510 (JP)**
• **SHUKE, Noriyuki**
**Kushiro-shi**
**Hokkaido 085-0062 (JP)**
• **NISHIKAWA, Kazuhiro**
**Nishinomiya-shi**
**Hyogo 662-0918 (JP)**
• **TAMAMURA, Naoyuki**
**Nishinomiya-shi**
**Hyogo 662-0918 (JP)**

(74) Representative: **Wibbelmann, Jobst**
**Wuesthoff & Wuesthoff**
**Patent- und Rechtsanwälte**
**Schweigerstrasse 2**
**81541 München (DE)**

(54) **CEREBRAL BLOOD FLOW QUANTIFICATION DEVICE, CEREBRAL BLOOD FLOW QUANTIFICATION METHOD AND PROGRAM**

(57)   A cerebral blood flow rate quantification device (10) comprises: a nuclear medicine image acquisition unit (12) for acquiring a nuclear medicine image of the head of a subject administered with a radiopharmaceutical; a count calculation unit (16) for calculating the number of counts obtained from a unit region indicating the same location in brain tissue in a plurality of nuclear medicine images of the head successively imaged between first time and second time; a cerebral blood flow rate calculation unit (24) for calculating, for each unit region, the value of a cerebral blood flow rate that makes the value of a function integrated from the first time to the second time equal to the number of counts, the function which, with cerebral blood flow rate and time elapsed from the administration of the radiopharmaceutical being as variables, expresses the concentration of the radiopharmaceutical in brain tissue; and a cerebral blood flow map generation unit (30) for generating a map showing cerebral blood flow based on the cerebral blood flow rate in each unit region. Consequently, the cerebral blood flow rate can be accurately quantified even at a relatively early stage after administration of a radiopharmaceutical.

【Fig.1】

EP 2 249 181 A1

**Description**

RELATED APPLICATIONS

This application claims the benefit of Japanese Patent Application No. 2008-031922 filed on February 13, 2008 in Japan, the contents of which are incorporated herein by reference.

TECHNICAL FIELD

The present invention relates to a device and method for quantifying the cerebral blood flow rate.

BACKGROUND ART

Cerebral vascular disorders, typified by cerebral infarction, rank high as causes of death, and sometimes leave serious aftereffects in cases of delayed discovery and delayed treatment. Early detection and diagnosis of the severity of cerebral vascular disorders are therefore very important.
As well as MRI and CT, nuclear medicine image diagnosis typified by SPECT or the like is known as a major diagnostic method for cerebral vascular disorders. Nuclear medicine image diagnosis can relatively easily quantify cerebral blood flow at the tissue level, and is therefore widely used for early diagnosis and diagnosis of the severity of cerebral vascular disorders. The microsphere method and the IMP-ARG method are mainly used as cerebral vascular disorder diagnosis that uses nuclear medicine image diagnosis.
The microsphere method regards a cerebral blood flow agent at an early stage after administration as a so-called microsphere, and based on a 1-compartment model determines a local cerebral blood flow rate from nuclear medicine image data. This method has an advantage of being theoretically simple and easy to calculate, but it has a disadvantage of being highly invasive due to its requirement for continuous arterial blood sampling. The method is also disadvantageous in that because agent washout from tissue to blood vessels is not considered, quantification accuracy decreases at a late stage after administration of the agent and the data collection time point is limited to a relatively early stage after administration.
The NIMS method has been suggested which estimates an input function using a time activity curve based on dynamic SPECT data of a breast instead of performing continuous arterial blood sampling, but it also is a method based on a 1-compartment model and cannot provide sufficient quantification accuracy at a late stage after administration of the agent.
The IMP-ARG method administers IMP (N-isopropyl-4-($^{123}$I)iodoamphetamine hydrochloride) as a radiopharmaceutical, substitutes the number of counts in a nuclear medicine image of a head after the elapse of a certain period of time into an equation of a 2-compartment model, and determines the cerebral blood flow rate using a lookup table method. An example of the IMP-ARG method is disclosed in Hidehiro Iida et al, "Quantitative mapping of regional cerebral blood flow using iodine-123-IMP and SPECT," the Journal of Nuclear Medicine, December 1994, Vol.35, No.12, 2019-2030. Based on a 2-compartment model, the IMP-ARG method is theoretically superior to the microsphere method, and can use a scheme in which one-point arterial blood sampling data is obtained to correct a standard input function when determining an input function to be used for calculation, so the method is less invasive than the microsphere method, which requires data of continuous blood sampling. The IMP-ARG method has these merits.

DISCLOSURE OF THE INVENTION

Problems to be solved by the invention

The conventional IMP-ARG method determines the cerebral blood flow rate using the number of counts at a time point when a particular time has passed since administration of an agent (hereinafter referred to as a "particular time point"). Due to a small amount of data in counts obtained from a nuclear medicine image acquired at a particular time point, it is difficult to accurately determine the cerebral blood flow rate. In order to obtain a sufficient number of counts, the conventional IMP-ARG method would collect data for a predetermined period of time (e.g. 20 to 30 minutes) centered at a particular time point and use the number of obtained counts as the number of counts at the particular time point. For example, in a case where the number of counts at a time point when 30 minutes has passed since administration of an agent were to be determined, counts would be collected for 20 minutes from a time point when 20 minutes has passed to a time point when 40 minutes has passed, and the number of the collected counts would be determined to be the number of counts at the time point when 30 minutes has passed.
This method, however, assumes that the number of counts at a particular time point is expressed by an average of the number of counts in a data collection time period, and therefore requires that temporal change in the number of counts should have converged in a data collection time period in order to accurately determine the cerebral blood flow rate. Due to a large temporal change in SPECT counts at an early stage after administration of a radiopharmaceutical, the conventional IMP-ARG method could not accurately quantify cerebral blood flow until the temporal change in the number of counts converges when a certain period of time has passed since administration of a radiopharmaceutical.

A purpose of the invention made in view of the above-mentioned circumstances is to provide a cerebral blood flow rate quantification device that can accurately quantify the cerebral blood flow rate at a relatively early stage after administration of a radiopharmaceutical as well as at a late stage after administration.

Means for solving the problems

A cerebral blood flow rate quantification device of the invention comprises: a nuclear medicine image acquisition unit for acquiring a nuclear medicine image of the head of a subject administered with a radiopharmaceutical; a count calculation unit for calculating the number of counts in the nuclear medicine image of the head continuously imaged between first time and second time, for each of unit regions forming the nuclear medicine image of the head; a cerebral blood flow rate calculation unit for calculating, for each of the unit regions, the value of a cerebral blood flow rate that makes the value of a function integrated from the first time to the second time equal to the number of counts calculated by the count calculation unit, the function including cerebral blood flow rate and time elapsed from the administration of the radiopharmaceutical as variables and expressing SPECT or PET counts corresponding to the concentration of the radiopharmaceutical in brain tissue; and a cerebral blood flow map generation unit for generating a map showing cerebral blood flow based on the cerebral blood flow rate in each of the unit regions.

There are other aspects of the invention as described below. This disclosure of the invention therefore intends to provide part of the aspects of the invention and does not intend to limit the scope of the invention described and claimed herein.

BRIEF DESCRIPTION OF THE DRAWINGS

Figure 1 shows a configuration of a cerebral blood flow rate quantification device of an embodiment;
Figure 2 shows an operation of the cerebral blood flow rate quantification device of the embodiment;
Figure 3 shows a configuration of a program used for cerebral blood flow rate quantification of the embodiment; and
Figure 4 shows an example of an image into which reconstruction images are added up.

BEST MODE OF EMBODYING THE INVENTION

The following is a detailed description of the invention. The embodiments described below are only examples of the invention, and the invention can be varied in various aspects. Therefore, the specific configurations and functions disclosed below do not limit the claims.

A cerebral blood flow rate quantification device of an embodiment comprises: a nuclear medicine image acquisition unit for acquiring a nuclear medicine image of the head of a subject administered with a radiopharmaceutical; a count calculation unit for calculating the number of counts in the nuclear medicine image of the head continuously imaged between first time and second time, for each of unit regions forming the nuclear medicine image of the head; a cerebral blood flow rate calculation unit for calculating, for each of the unit regions, the value of a cerebral blood flow rate that makes the value of a function integrated from the first time to the second time equal to the number of counts calculated by the count calculation unit, the function including cerebral blood flow rate and time elapsed from the administration of the radiopharmaceutical as variables and expressing SPECT or PET counts corresponding to the concentration of the radiopharmaceutical in brain tissue; and a cerebral blood flow map generation unit for generating a map showing cerebral blood flow based on the cerebral blood flow rate in each of the unit regions.

In this configuration, the cerebral blood flow rate can be appropriately determined even if temporal change in the number of collected counts has not converged, and the cerebral blood flow rate can be accurately quantified even at an early stage after administration of a radiopharmaceutical. In the embodiment, the cerebral blood flow rate is quantified not based on the concentration of a radiopharmaceutical at a particular time point but with consideration for a change in the concentration of a radiopharmaceutical within a predetermined duration, and therefore the cerebral blood flow rate can be appropriately determined even if the number of counts varies during the duration. Nuclear medicine images of a head to be used for calculation of the number of counts may be obtained by picking up nuclear medicine images of the head between the first and second time from among nuclear medicine images of the head successively imaged immediately after administration of a radiopharmaceutical, or by imaging nuclear medicine images of the head only between the first and second time.

In the cerebral blood flow rate quantification device of the embodiment, the nuclear medicine image acquisition unit may acquire a plurality of nuclear medicine images of the head between the first time and the second time, and the count calculation unit may add up the number of counts obtained from the unit region indicating the same location in the brain tissue in the plurality of nuclear medicine images of the head imaged between the first time and the second time to calculate the number of counts for each of the unit regions.

This configuration allows the number of counts to be appropriately determined for each of the unit regions by using, for example, a plurality of nuclear medicine images of a head acquired with dynamic scanning.

The cerebral blood flow rate quantification device of the embodiment may have an image selector for, in order to designate successive time series nuclear medicine images of the head to be used to quantify the cerebral blood flow rate, selecting first and last images of the plurality of successive nuclear medicine images of the head from among the plurality of nuclear medicine images of the head acquired by the nuclear medicine image acquisition unit, where the count calculation unit may determine imaging start time of the first nuclear medicine image of the head to be the first time and determine imaging finish time of the last nuclear medicine image of the head to be the second time to calculate the number of counts. The cerebral blood flow rate quantification device of the embodiment may have a time information input unit for receiving an input of time information, where the count calculation unit may determine the first and second time based on the inputted time information to calculate the number of counts.

In this configuration, data to be used to quantify the cerebral blood flow rate can be set appropriately.

In the cerebral blood flow rate quantification device of the embodiment, the cerebral blood flow rate calculation unit may use an equation (1) as the function, to calculate the cerebral blood flow rate $k_1$ in each of the unit regions:

[Mathematical expression 1]

$$\int_{t_1}^{t_2} B(t)\,dt = \int_{t_1}^{t_2} k_1 \int_0^t C(\tau)\cdot e^{-\frac{k_1}{V_d}\cdot(t-\tau)}\,d\tau\,dt \qquad (1)$$

where $B(t)$: number of counts, $k_1$: cerebral blood flow rate, $C(\tau)$: input function, $V_d$: partition coefficient between brain tissue and blood, $t_1$: first time, and $t_2$: second time.

In this configuration, a conventional 2-compartment model can be used, and therefore parameters and an estimation method for the input function according to known methods can be used.

In the cerebral blood flow rate quantification device of the embodiment, the cerebral blood flow rate calculation unit may have a table creation unit for creating a table showing cerebral blood flow rates corresponding to all of the calculated numbers of counts ranging from a minimum number of counts to a maximum number of counts, and the cerebral blood flow rate corresponding to the number of counts in each of the unit regions may be determined with reference to the table.

In this configuration, once the table is created, the cerebral blood flow rate can be determined with reference to the table. When there are many unit regions to be calculated, the calculation efficiency can be enhanced by creating the table. Since the table has data on cerebral blood flow rates corresponding to all of the calculated numbers of counts ranging from a minimum number of counts to a maximum number of counts, any number of counts has a corresponding cerebral blood flow rate and no interpolation process is required, so the cerebral blood flow rate can be appropriately determined.

A cerebral blood flow rate quantification method of the embodiment is for a computer to quantify a cerebral blood flow rate based on a nuclear medicine image of the head of a subject administered with a radiopharmaceutical, and the cerebral blood flow rate quantification method comprises the steps of: the computer acquiring a nuclear medicine image of the head of a subject administered with a radiopharmaceutical; the computer calculating the number of counts in the nuclear medicine image of the head continuously imaged between first time and second time, for each of unit regions forming the nuclear medicine image of the head; the computer calculating, for each of the unit regions, the value of a cerebral blood flow rate that makes the value of a function integrated from the first time to the second time equal to the number of counts calculated in the step of calculating the number of counts, the function including cerebral blood flow rate and time elapsed from the administration of the radiopharmaceutical as variables and expressing SPECT or PET counts corresponding to the concentration of the radiopharmaceutical in brain tissue; and the computer generating a map showing cerebral blood flow based on the cerebral blood flow rate in each of the unit regions.

In this configuration, as in the case of the cerebral blood flow rate quantification device of the embodiment, the cerebral blood flow rate can be appropriately determined even if temporal change in the number of collected counts has not converged, and the cerebral blood flow rate can be accurately quantified even at an early stage after administration of a radiopharmaceutical. The various configurations of the cerebral blood flow rate quantification device of the embodiment can also be applied to the cerebral blood flow rate quantification method of the embodiment.

A program of the embodiment is for quantifying a cerebral blood flow rate based on a nuclear medicine image of the head of a subject administered with a radiopharmaceutical, and the program causes a computer to execute the steps of: acquiring a nuclear medicine image of the head of a subject administered with a radiopharmaceutical; calculating the number of counts in the nuclear medicine image of the head continuously imaged between first time and second time, for each of unit regions forming the nuclear medicine image of the head; calculating, for each of the unit regions, the value of a cerebral blood flow rate that makes the value of a function integrated from the first time to the second time equal to the number of counts calculated in the step of calculating the number of counts, the function which including cerebral blood flow rate and time elapsed from the administration of the radiopharmaceutical as variables and expressing SPECT or PET counts corresponding to the concentration of the radiopharmaceutical in brain tissue; and generating a map showing cerebral blood flow based on the cerebral blood flow rate in each of the unit regions.

In this configuration, as in the case of the cerebral blood flow rate quantification device of the embodiment, the cerebral blood flow rate can be appropriately determined even if temporal change in the number of collected counts has not converged, and the cerebral blood flow rate can be accurately quantified even at an early stage after administration of a radiopharmaceutical. The various configurations of the cerebral blood flow rate quantification device of the embodiment can also be applied to the program of the embodiment.

Now, the cerebral blood flow rate quantification device and cerebral blood flow rate quantification method of an embodiment of the invention will be described with reference to the drawings.

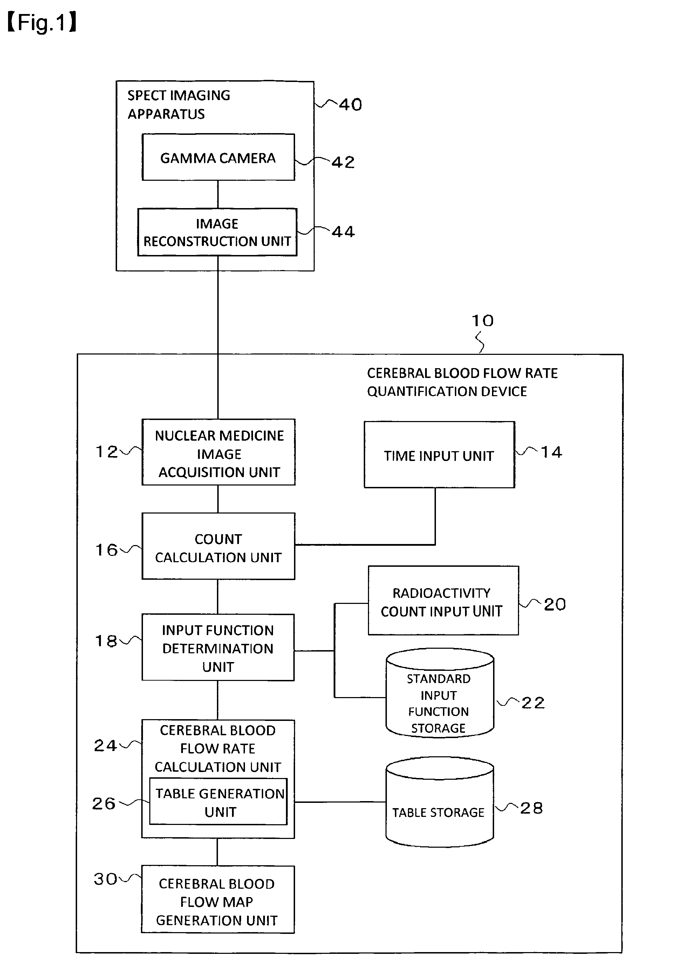

Figure 1 shows a configuration of the cerebral blood flow rate quantification device 10 of the embodiment. The cerebral blood flow rate quantification device 10 has a nuclear medicine image acquisition unit 12 for acquiring a reconstruction image inputted from a SPECT imaging apparatus 40. In a preferred aspect, the SPECT imaging apparatus 40 continuously images a subject with a gamma camera 42 from when the subject is administered with a radiopharmaceutical, and generates a reconstruction image from a projection image with an image reconstruction unit 44. The nuclear medicine image acquisition unit 12 successively acquires reconstruction images obtained by the SPECT imaging apparatus 40. The cerebral blood flow rate quantification device 10 uses the reconstruction images obtained from the SPECT imaging apparatus 40 to quantify the cerebral blood flow rate.

The cerebral blood flow rate quantification device 10 has: a time input unit 14 for inputting first time and second time for designating nuclear medicine images of a head to be used for the quantification; and a count calculation unit 16 for determining the number of counts for each of pixels in a region of interest whose cerebral blood flow is to be determined, with the use of a plurality of reconstruction images imaged between the first and second time. In a preferred aspect, the count calculation unit 16 adds up the number of counts obtained from a pixel indicating the same location in brain tissue in the plurality of reconstruction images to calculate the number of counts to be used to calculate the cerebral blood flow rate. In the embodiment, a unit region indicating the same location in brain tissue is defined by one pixel. The count calculation unit 16 may also add up a plurality of reconstruction images to generate an addition image and determine the number of counts for each pixel in the addition image. A unit region indicating the same location in brain tissue does not necessarily require to be formed of one pixel, but may be formed of e.g. $2 \times 2$, four, pixels. However, the smaller a unit region is, the higher the spatial resolution of the cerebral blood flow map is.

The cerebral blood flow rate quantification device 10 has an input function determination unit 18 for determining an input function for a subject. The input function here is a function expressing a temporal change in the amount of a radiopharmaceutical transferring to brain tissue of a subject. The input function determination unit 18 is connected with a radioactivity count input unit 20 and a standard input function storage 22. The standard input function storage 22 stores a previously created standard input function. The radioactivity count input unit 20 receives an input of radioactivity counts in blood sampled from a subject at a predetermined time after administration of an agent (e.g. eight minutes after administration of an agent). The input function determination unit 18 corrects the standard input function with radioactivity counts inputted from the radioactivity count input unit 20 to determine the input function.

The cerebral blood flow rate quantification device 10 has a cerebral blood flow rate calculation unit 24 for calculating the cerebral blood flow rate with the use of a function expressing SPECT or PET counts corresponding to the concentration of a radiopharmaceutical in brain tissue. The concentration of a radiopharmaceutical (the number of counts) here is expressed by using the input function by the following equation (1):

[Mathematical expression 2]

$$\int_{t_1}^{t_2} B(t)dt = \int_{t_1}^{t_2} k_1 \int_0^t C(\tau) \cdot e^{-\frac{k_1}{V_d} \cdot (t-\tau)} d\tau dt \qquad (1)$$

where B(t): number of counts, $k_1$: cerebral blood flow rate, C($\tau$): input function, $V_d$: partition coefficient between brain tissue and blood, $t_1$: first time, and $t_2$: second time.

The cerebral blood flow rate calculation unit 24 substitutes the number of counts in each pixel determined by the count calculation unit 16 into the left side of the equation (1). The cerebral blood flow rate calculation unit 24 then performs fitting according to a known method to determine the cerebral blood flow rate $k_1$ that makes the equation (1) true. The cerebral blood flow rate in a region indicated by each pixel can thus be determined. At this time, the value of the partition coefficient $V_d$ determined in advance by using a known method or the like may be substituted into the above equation (1) before the fitting. As a result, the number of parameters to be used for the fitting can be reduced and the accuracy can be improved.

The cerebral blood flow rate calculation unit 24 has a table generation unit 26 for reducing the load of the calculation process. The table generation unit 26 has a function of generating a table showing the cerebral blood flow rates $k_1$ corresponding to all numbers of counts ranging from a minimum number of counts to a maximum number of counts calculated by the count calculation unit 16. Specifically, for example, suppose a minimum number of counts obtained from each pixel is 1 and a maximum number of counts is 1000; and the cerebral blood flow rate $k_1(1)$ for when 1 is substituted into the left side of the equation (1) (which corresponds to the number of counts being 1), the cerebral blood flow rate $k_1(2)$ for when 2 is substituted into the left side of the equation (1) (which corresponds to the number of counts being 2), ... , and the cerebral blood flow rate $k_1(1000)$ for when 1000 is substituted into the left side of the equation (1) (which corresponds to the number of counts being 1000) are in advance determined and stored in a table storage 28. The cerebral blood flow rate calculation unit 24 then reads from the table 28 the cerebral blood flow rate $k_1(n)$ corresponding to the number of counts being n in each pixel calculated by the count calculation unit 16. Consequently, when the number of pixels the cerebral blood flow rate in which is to be determined is more than 1000 pixels, the cerebral blood flow rate can be determined efficiently by reducing the number of the fitting calculations.

The cerebral blood flow rate quantification device 10 has a cerebral blood flow map generation unit 30 for generating a cerebral blood flow map with the use of a known method, based on the cerebral blood flow rate in each pixel determined by the cerebral blood flow rate calculation unit 24. In a preferred aspect, the cerebral blood flow map generation unit 30 generates a map showing the cerebral blood flow rate in colors.

An operation of the cerebral blood flow rate quantification device 10 of the embodiment will next be described. In a preferred aspect, when the cerebral blood flow rate is quantified, a subject is administered with $^{123}$I-IMP or other radiopharmaceutical, and a SPECT image of the head of the subject is imaged from the time of administration by the SPECT imaging apparatus 40. The cerebral blood flow rate quantification device 10 uses a reconstruction image of the SPECT image to quantify the cerebral blood flow rate.

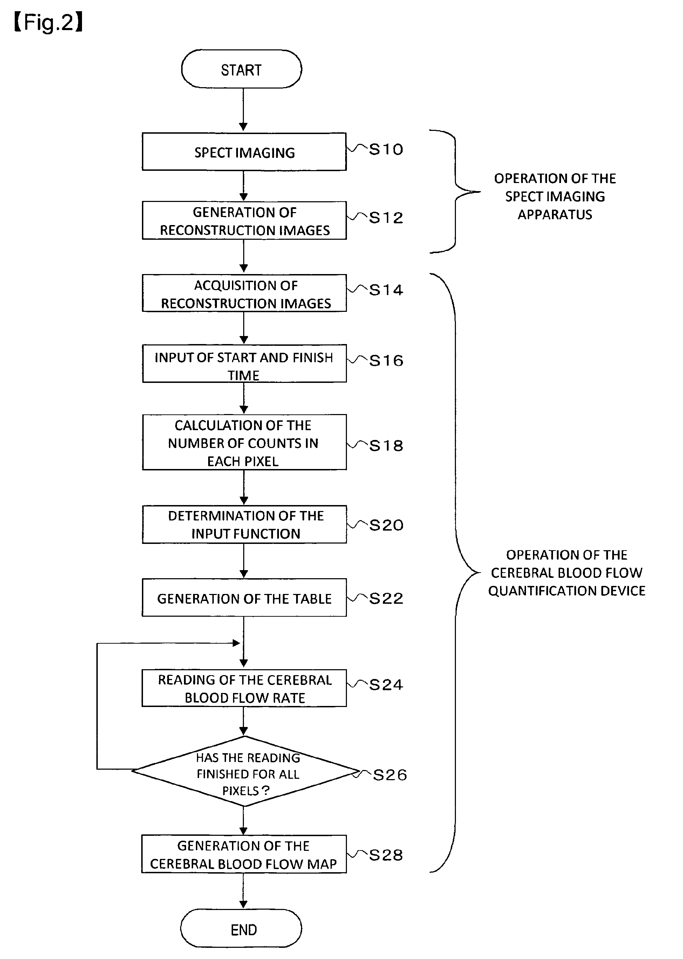

Figure 2 is a flowchart showing an operation of the cerebral blood flow rate quantification device 10 of the embodiment. First, the SPECT imaging apparatus 40 images with a known method the head of a subject administered with an agent (S10). The SPECT imaging apparatus 40 reconstructs the projection image obtained by the imaging to generate a reconstruction image (S12), and transmits the generated reconstruction image to the cerebral blood flow rate quantification device 10. In the following description, when a plurality of temporally successive reconstruction images are imaged, a process to acquire each reconstruction image is called a "phase." One phase ends, for example, in about 2.5 minutes.

The cerebral blood flow rate quantification device 10 acquires the reconstruction image transmitted from the SPECT imaging apparatus 40 (S14). In a preferred aspect, the cerebral blood flow rate quantification device 10 induces a user to input first time t1 and second time t2, and receives the input of the first time t1 and second time t2 from the user (S16). The cerebral blood flow rate quantification device 10 identifies the phase that includes the first time t1 in its imaging time period, and determines that the phase next to that phase is to be the first phase. The cerebral blood flow rate quantification device 10 also identifies the phase that includes the second time in its imaging time period, and determines that the phase immediately preceding that phase is to be the last phase. The cerebral blood flow rate quantification device 10 then selects a plurality of reconstruction images successively imaged between the first phase and the last phase. While the phase next to a phase that includes inputted first time t1 is determined to be the first phase here, the phase that includes the first time t1 may be determined to be the first phase. Similarly, a phase that includes inputted second time t2 may be determined to be the last phase.

The cerebral blood flow rate quantification device 10 replaces the inputted first time t1 by imaging start time of the above-selected first phase, and replaces the inputted second time t2 by imaging finish time of the above-selected last phase. This replacement is a process to match, in the above-described equation (1), the first time t1 and second time t2 to be substituted into the right side with the period of time during which images to be used for the calculation of the number of counts to be substituted into the left side are acquired.

The cerebral blood flow rate quantification device 10 adds up, for each pixel in reconstruction images, the number of counts in a pixel indicating the same location in brain tissue in a reconstruction image imaged in each phase included between the first phase and the last phase, and calculates for each pixel the number of counts to be used for the quantification. That is, the cerebral blood flow rate quantification device 10 adds up the number of counts obtained from a pixel indicating the same part in a reconstruction image obtained in each phase included between the first and last phases selected above (S18).

The cerebral blood flow rate quantification device 10 then determines the input function of the radiopharmaceutical for brain tissue of the subject (S20). In a preferred aspect, the cerebral blood flow rate quantification device 10 uses radioactivity counts determined for arterial blood sampled from the subject, to correct the standard input function and determine the input function specific to the subject. The cerebral blood flow rate quantification device 10 then uses the function indicating the concentration of the radiopharmaceutical shown in the above-described equation (1) to generate a table that shows the cerebral blood flow rate $k_1$ corresponding to:

[Mathematical expression 3]

$$\int_{t_1}^{t_2} B(t)\,dt$$

(which corresponds to the number of counts determined in the above step S16) (S22). A specific method of creating the table is as already described. The cerebral blood flow rate quantification device 10 reads from the table 28 the cerebral blood flow rate corresponding to the number of counts in each pixel determined in the above step S16 (S24).

The cerebral blood flow rate quantification device 10 judges whether it has read the cerebral blood flow rate corresponding to the number of counts for all pixels in a region the cerebral blood flow rate in which is desired to be determined or not (S26), and if it has not finished the reading from all pixels (No at S26), it reads the cerebral blood flow rate for the rest of the pixels. If the cerebral blood flow rate quantification device 10 has finished the reading from all pixels (Yes at S26), it uses the read cerebral blood flow rate in each pixel to generate a cerebral blood flow map (S28). The cerebral blood flow map can be generated, for example, by a method in which brightness, color, or the like corresponding to the cerebral blood flow rate determined for each pixel is displayed in an arrangement corresponding to each pixel.

Up to this point, the cerebral blood flow rate quantification device 10 and cerebral blood flow rate quantification method of the embodiment have been described.

The cerebral blood flow rate quantification device 10 of the embodiment determines the cerebral blood flow rate that makes the value of the function, which expresses SPECT or PET counts corresponding to the concentration of the radiopharmaceutical, integrated from the first time t1 to the second time t2 equal to the number of counts in reconstruction images successively imaged from the first time t1 to the second time t2, and can therefore determine the cerebral blood flow rate appropriately even when there is a temporal change in the number of counts between the first time t1 and the second time t2. As a result, the cerebral blood flow rate can be quantified at an early stage after administration of an agent, and the time required for quantifying cerebral blood flow can be shortened. This is very effective because the shortening in the quantification time allows the burden on a subject to be reduced and leads to an increase in the number of examinations in examination facilities.

While a cerebral blood flow rate quantification device of the invention has been described in detail with the most preferred embodiment, the invention is not limited to the above-described embodiment.

For example, PET images can also be used instead of SPECT images. In this case, a process similar to the above may be performed with an agent for PET examinations used as the radiopharmaceutical to be administered to a subject, and with the SPECT apparatus in Figure 1 and the SPECT imaging in Figure 2 replaced by a PET apparatus and PET imaging, respectively.

While there has been described a configuration of the cerebral blood flow rate quantification device 10 of the above-described embodiment, each component of the cerebral blood flow rate quantification device 10 shown in Figure 1 may be realized by a computer executing a program for quantifying the cerebral blood flow rate.

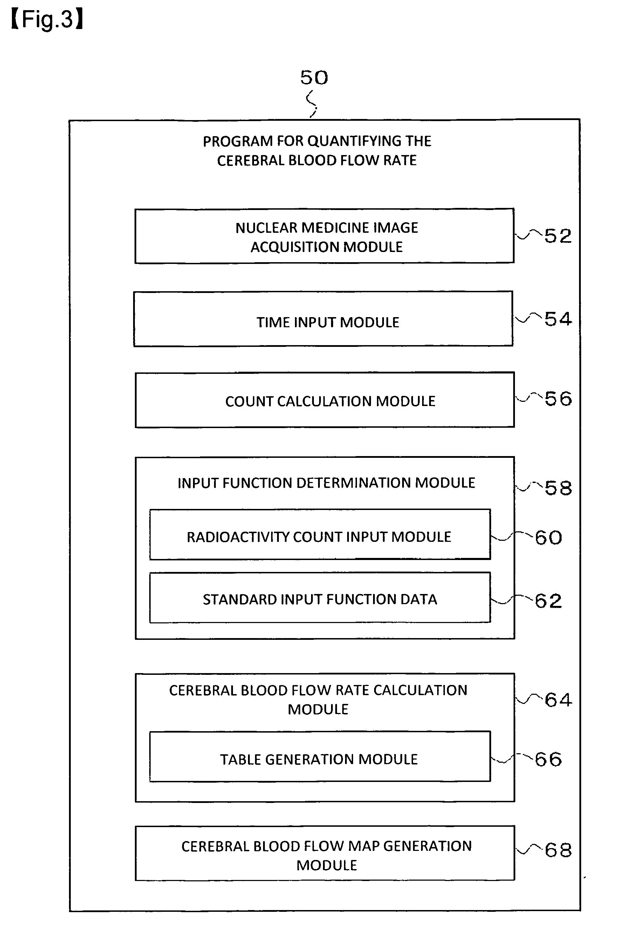

Figure 3 shows a configuration of a program for quantifying the cerebral blood flow rate. The program for quantifying the cerebral blood flow rate 50 has a nuclear medicine image acquisition module 52, a time input module 54, a count calculation module 56, an input function determination module 58, a cerebral blood flow rate calculation module 64, and a cerebral blood flow map generation module 68. The input function determination module 58 has a radioactivity count input module 60 and standard input function data 62, and the cerebral blood flow rate calculation module 64 has a table generation module 66.

The nuclear medicine image acquisition unit 12 shown in Figure 1 can be realized by a computer executing the nuclear medicine image acquisition module 52. Likewise, the time input unit 14, count calculation unit 16, input function determination unit 18, radioactivity count input unit 20, cerebral blood flow rate calculation unit 24, table generation unit 26, and cerebral blood flow map generation unit 30 shown in Figure 1 can be realized by a computer executing the time input module 54, count calculation module 56, input function determination module 58, radioactivity count input module 60, cerebral blood flow rate calculation module 64, table generation module 66, and cerebral blood flow map generation module 68. The standard input function data 62 included in the input function determination module 58 corresponds to the standard input function storage 22 shown in Figure 1.

There has been described in the above-described embodiment an example where the first time t1 and the second time t2 are inputted to designate nuclear medicine images of a head to be used for quantification, but images to be used to quantify the cerebral blood flow rate, the first time t1, and the second time t2 may be determined by selecting reconstruction images from a series of successively imaged reconstruction images. For example, reconstruction images acquired by the nuclear medicine image acquisition unit 12 are displayed in a time series, and a user is asked about which reconstruction images are to be used to quantify the cerebral blood flow rate. The user selects the first and last images of a plurality of reconstruction images to be used to quantify the cerebral blood flow rate. The cerebral blood flow rate quantification device determines the number of counts from the selected two reconstruction images and images situated temporally between the two reconstruction images. The cerebral blood flow rate quantification device then determines imaging start time of the selected first reconstruction image to be the first time t1 and determines imaging finish time of the last reconstruction image to be the second time t2, to quantify the cerebral blood flow rate using the above-described equation (1).

The cerebral blood flow rate quantification device 10 may determine the first time t1 and the second time t2 by some kind of criterion. For example, a time point when an average of the number of counts for all pixels in reconstruction images becomes a predetermined threshold or higher may be determined to be the first time t1, and a time point a certain period of time after the first time t1 may be determined to be the second time t2.

While in the above-described embodiment the equation (1) expressing the concentration of a radiopharmaceutical is a function based on a 2-compartment model, a function based on a model other than 2-compartment models may also be used in the invention.

There has been described in the above-described embodiment an example where a standard input function is corrected based on radioactivity counts inputted by the radioactivity count input unit 20, but an input function estimated based on another known method may also be used.

There has been described in the above-described embodiment an example where the cerebral blood flow rate calculation unit 24 generates a table showing the cerebral blood flow rates corresponding to the numbers of counts in order to reduce the load of the calculation process, but the table does not necessarily require to be generated if the number of pixels the cerebral blood flow rate in which is to be determined is not many or if a computer having a high computing performance is used.

There has been described in the above-described embodiment an example where: time between the first time t1 and the second time t2 is divided into a plurality of phases; a reconstruction image is generated for each phase; and the plurality of generated nuclear medicine images of a head are used to quantify the cerebral blood flow rate, but the plurality of nuclear medicine images of the head does not necessarily require to be used. For example, one reconstruction image may be reconstructed from a plurality of projection images obtained between the first time t1 and the second time t2, and the number of counts in the reconstruction image may be used to quantify the cerebral blood flow rate. Since the total number of counts to be obtained from nuclear medicine images of the head is the same regardless of whether time between the first time t1 and the second time t2 is divided into a plurality of phases or not, the cerebral blood flow rate can be appropriately quantified as with the embodiment even when one reconstruction image is used.

There has been described in the above-described embodiment a configuration where the count calculation unit 16, based on the first time t1 and second time t2 inputted from the time input unit 14, determines images to be used to quantify the cerebral blood flow rate from among reconstruction images acquired by the nuclear medicine image acquisition unit 12, but a configuration may be adopted in which an input from the time input unit 14 is inputted to the nuclear medicine image acquisition unit 12 and the nuclear medicine image acquisition unit 12 acquires reconstruction images only between the first time t1 and the second time t2. In this case, the nuclear medicine image acquisition unit 12 may give information on the first time t1 and second time t2 to the SPECT imaging apparatus 40, and the SPECT imaging apparatus 40 may be controlled to image only between the first time and the second time.

Examples

Working examples and a comparative example will be shown below to describe the invention in further detail, but the invention is not limited to the following. In the working examples, regions of interest were set in reconstruction images, and the cerebral blood flow rate in the set regions of interest was quantified on the images by using reconstruction images imaged in the following time: (Example 1) 0-10 minutes; (Example 2) 10-20 minutes; (Example 3) 0-20 minutes; (Example 4) 15-35 minutes; and (Example 5) 15-25 minutes. As a comparative example, the cerebral blood flow rate in the regions of interest was quantified by a conventional method by using reconstruction images imaged in 15-35 minutes after administration of an agent.

[Collection of SPECT data]

A subject was administered with $^{123}$I-IMP (200 MBq), and 14 successive scans of dynamic SPECT data were collected at 2.5 minutes per scan.

[Correction of a standard input function]

An input function was determined by using radioactivity counts in arterial blood of the subject and correcting a standard input function created by Research Institute for Brain and Blood Vessels Akita (see Non-patent document 1) in the following manner: Arterial blood was sampled eight minutes after the administration of the radiopharmaceutical, the radioactivity count was measured with a scintillation counter, and the radioactivity count per unit time and unit mass was determined; the obtained radioactivity count per unit time and unit mass was divided by the radioactivity count at the eight minute point of the standard input function to determine a correction factor; and the standard input function was multiplied by the obtained correction factor so as to be corrected, and was further corrected with a cross calibration factor to determine the input function $C(\tau)$.

The correction using a cross calibration factor is described in: Hidehiro Iida et al., "A method to quantitate cerebral blood flow using a rotating gamma camera and iodine-123 iodoamphetamine with one blood sampling," European Journal of Nuclear Medicine, October 1994, Vol. 21, No. 10, 1072-1084; Takenori Hachiya et al., "Stability of cross calibration between SPECT apparatuses and well counters (SPECT souchi to well counter no cross calibration no anteisei)," the Japanese Journal of Nuclear Medicine Technology, 1995, Vol. 15, No. 2, 105-109; and Takenori Hachiya et al., "Source Error Factors in Cross Calibration for Quantitative Single Photon Emission Computed Tomography," the Japanese Journal of Nuclear Medicine Technology, 1996, Vol. 16, No. 2, 58-62.

[Examples 1 to 5] Calculation of the cerebral blood flow rate in the working examples (Creation of tables)

In the working examples according to the invention, the above-described equation (1) was used as the function expressing the concentration of the radiopharmaceutical. In Example 1, the first time t1 was 0 minute, the second time t2 was 10 minutes, and the left side was substituted with 1, in the equation (1); and then fitting was performed according to the Gauss-Newton algorism to calculate the value of $k_1$. This operation was successively performed with the left side substituted with integers ranging from 2 to 1000, and Table 1 showing $k_1$ versus an arbitrary number of counts was created.

The same operation as described above was performed: in Example 2 with the first time t1 being 10 minutes and the second time t2 being 20 minutes; in Example 3 with the first time t1 being 0 minute and the second time t2 being 20 minutes; in Example 4 with the first time t1 being 15 minutes and the second time t2 being 35 minutes; and in Example 5 with the first time t1 being 15 minutes and the second time t2 being 25 minutes, and a table corresponding to each working example was created. They are Table 2, Table 3, Table 4, and Table 5, respectively. In the creation of each table, 35 (mUmL) was used as the value of $V_d$.

(Quantification of the cerebral blood flow rate)

Image reconstruction according to a filtered back projection algorithm was performed for each phase in each working example to create a plurality of transverse images (reconstruction images of matrix: $64 \times 64$ and pixel size: 3.44 mm $\times$ 3.44 mm $\times$ 3.44 mm). A slice at the basal ganglia level was selected for each of: four phases ranging from 0 to 10 minutes in Example 1; four phases ranging from 10 to 20 minutes in Example 2; eight phases ranging from 0 to 20 minutes in Example 3; eight phases ranging from 15 to 35 minutes in Example 4; and four phases ranging from 15 to 25 minutes in Example 5, and the selected slices were added up to create addition images (referred to as Addition images 1 to 5, respectively).

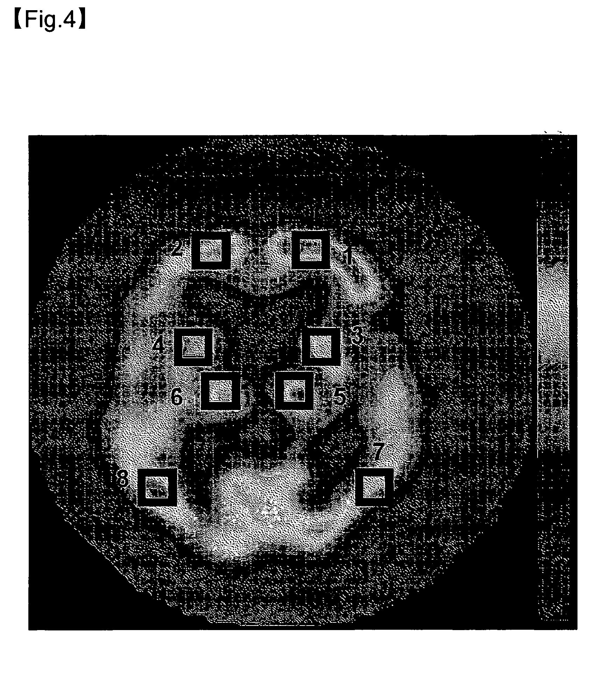

Figure 4 shows one example of the addition images. As shown in Figure 4, square (5 pixels $\times$ 5 pixels) regions of interest were set in eight places in the addition image. The blood flow rate was determined for each pixel in created Addition images 1 to 5 with the use of Tables 1 to 5 created as above, respectively, to create a CBF map. The cerebral blood flow rate (mUmin/100g) in each pixel included within the regions of interest was read from the obtained CBF map, and an average value of the cerebral blood flow rate was calculated for each region of interest.

[Comparative example] Calculation of the cerebral blood flow rate according to a conventional method

As with the above-described working examples, a plurality of reconstruction images were created for each SPECT acquisition data of a total of eight phases ranging from 15 minutes to 35 minutes after the administration. A slice at the basal ganglia level was selected for each phase, and they were added up to create an addition image (see Figure 4). Square (5 pixels $\times$ 5 pixels) regions of interest were set in the eight places shown in Figure 4. Separately, a table showing the value of $k_1$ corresponding to B(t) was created by means of a method according to Non-patent document 1, by using an equation (2) below. By using the above table, $k_1$ corresponding to B(t), the number of counts in each pixel in the addition image determined as above, was determined to create a CBF map. Then, $k_1$ in pixels included within the above regions of interest was read from the obtained CBF map, and an average value was calculated for each region of interest. In the equation (2), 35 (mUmL) was used as the value of $V_d$.

[Mathematical expression 4]

$$B(t) = k_1 \int_0^t C(\tau) \cdot e^{-\frac{k_1}{V_d}(t-\tau)} d\tau \quad (2)$$

where B(t): number of counts, $k_1$: cerebral blood flow rate, $C(\tau)$: input function, $V_d$: partition coefficient between brain tissue and blood, and t: time elapsed to a particular time point.

[Result]

The result is shown in Result table 1. Calculated values of the cerebral blood flow rate in all the regions of interest indicated almost the same value regardless of period used for the quantification, and also indicated almost the same value as those determined by the conventional method. This result indicated that the quantification method according to the present method could provide almost the same value as that determined by the conventional method, regardless of time when nuclear medicine images of a head to be used for the quantification were obtained.

[Result table 1]

|  | Quantification time (time after administration, min) | | | | | |
|---|---|---|---|---|---|---|
|  | Conventional method (Comparative example) | 0-10 min (Example 1) | 10-20 min (Example 2) | 0-20 min (Example 3) | 15-35 min (Example 4) | 15-25 min (Example 5) |
| ROI 1 | 31.6 | 33.1 | 31.8 | 32.2 | 32.1 | 32.8 |
| ROI 2 | 29.4 | 30.1 | 30.2 | 30.2 | 29.8 | 30.2 |
| ROI 3 | 29.9 | 29.4 | 31.2 | 30.4 | 30.4 | 32.7 |
| ROI 4 | 27.1 | 25.5 | 26.6 | 26.1 | 27.5 | 27.2 |
| ROI 5 | 31.1 | 30.0 | 33.0 | 31.8 | 31.5 | 33.2 |
| ROI 6 | 30.0 | 32.2 | 29.3 | 30.4 | 30.5 | 30.9 |
| ROI 7 | 27.3 | 28.3 | 27.0 | 27.5 | 27.7 | 25.6 |
| ROI 8 | 33.8 | 32.1 | 32.6 | 32.3 | 34.3 | 32.4 |

While there have been described what are at present considered to be preferred embodiments of the invention, it will be understood that various modifications and variations may be made thereto, and it is intended that appended claims cover all such modifications and variations as fall within the true spirit and scope of the invention.

Industrial applicability

The invention has an advantage of being able to quantify the cerebral blood flow rate at an early stage after administration of a radiopharmaceutical, and is useful as an apparatus for quantifying the cerebral blood flow rate, and the like.

**Claims**

1. A cerebral blood flow rate quantification device comprising:

   a nuclear medicine image acquisition unit for acquiring a nuclear medicine image of the head of a subject administered with a radiopharmaceutical;
   a count calculation unit for calculating the number of counts in the nuclear medicine image of the head continuously imaged between first time and second time, for each of unit regions forming the nuclear medicine image of the head;
   a cerebral blood flow rate calculation unit for calculating, for each of the unit regions, the value of a cerebral blood flow rate that makes the value of a function integrated from the first time to the second time equal to the number of counts calculated by the count calculation unit, the function including cerebral blood flow rate and time elapsed from the administration of the radiopharmaceutical as variables and expressing SPECT or PET counts corresponding to the concentration of the radiopharmaceutical in brain tissue; and
   a cerebral blood flow map generation unit for generating a map showing cerebral blood flow based on the cerebral blood flow rate in each of the unit regions.

2. The cerebral blood flow rate quantification device according to claim 1,
   wherein the nuclear medicine image acquisition unit acquires a plurality of nuclear medicine images of the head between the first time and the second time, and
   wherein the count calculation unit adds up the number of counts obtained from the unit region indicating the same location in the brain tissue in the plurality of nuclear medicine images of the head imaged between the first time and the second time to calculate the number of counts for each of the unit regions.

3. The cerebral blood flow rate quantification device according to claim 2, having an image selector for, in order to designate successive time series nuclear medicine images of the head to be used to quantify the cerebral blood flow rate, selecting first and last images of the plurality of successive nuclear medicine images of the head from among the plurality of nuclear medicine images of the head acquired by the nuclear medicine image acquisition unit, wherein the count calculation unit determines imaging start time of the first nuclear medicine image of the head to be the first time and determines imaging finish time of the last nuclear medicine image of the head to be the second time to calculate the number of counts.

4. The cerebral blood flow rate quantification device according to claim 1, having a time information input unit for receiving an input of time information,
   wherein the count calculation unit determines the first and second time based on the inputted time information to calculate the number of counts.

5. The cerebral blood flow rate quantification device according to any one of claims 1 to 4, wherein the cerebral blood flow rate calculation unit uses an equation (1) as the function, to calculate the cerebral blood flow rate $k_1$ in each of the unit regions:

   [Mathematical expression 1]

$$\int_{t_1}^{t_2} B(t)\,dt = \int_{t_1}^{t_2} k_1 \int_0^t C(\tau) \cdot e^{-\frac{k_1}{V_d} \cdot (t-\tau)}\, d\tau\, dt \quad (1)$$

   where B(t): number of counts, $k_1$: cerebral blood flow rate, $C(\tau)$: input function, $V_d$: partition coefficient between brain tissue and blood, $t_1$: first time, and $t_2$: second time.

6. The cerebral blood flow rate quantification device according to any one of claims 1 to 5,
   wherein the cerebral blood flow rate calculation unit has a table creation unit for creating a table showing cerebral blood flow rates corresponding to all of the calculated numbers of counts ranging from a minimum number of counts to a maximum number of counts, and
   wherein the cerebral blood flow rate corresponding to the number of counts in each of the unit regions is determined with reference to the table.

**7.** A method for a computer to quantify a cerebral blood flow rate based on a nuclear medicine image of the head of a subject administered with a radiopharmaceutical, the cerebral blood flow rate quantification method comprising the steps of:

the computer acquiring a nuclear medicine image of the head of a subject administered with a radiopharmaceutical;

the computer calculating the number of counts in the nuclear medicine image of the head continuously imaged between first time and second time, for each of unit regions forming the nuclear medicine image of the head;

the computer calculating, for each of the unit regions, the value of a cerebral blood flow rate that makes the value of a function integrated from the first time to the second time equal to the number of counts calculated in the step of calculating the number of counts, the function including cerebral blood flow rate and time elapsed from the administration of the radiopharmaceutical as variables and expressing SPECT or PET counts corresponding to the concentration of the radiopharmaceutical in brain tissue; and

the computer generating a map showing cerebral blood flow based on the cerebral blood flow rate in each of the unit regions.

**8.** A program for quantifying a cerebral blood flow rate based on a nuclear medicine image of the head of a subject administered with a radiopharmaceutical, the program causing a computer to execute the steps of:

acquiring a nuclear medicine image of the head of a subject administered with a radiopharmaceutical;

calculating the number of counts in the nuclear medicine image of the head continuously imaged between first time and second time, for each of unit regions forming the nuclear medicine image of the head;

calculating, for each of the unit regions, the value of a cerebral blood flow rate that makes the value of a function integrated from the first time to the second time equal to the number of counts calculated in the step of calculating the number of counts, the function including cerebral blood flow rate and time elapsed from the administration of the radiopharmaceutical as variables and expressing SPECT or PET counts corresponding to the concentration of the radiopharmaceutical in brain tissue; and

generating a map showing cerebral blood flow based on the cerebral blood flow rate in each of the unit regions.

【Fig.1】

SPECT IMAGING APPARATUS ~40

GAMMA CAMERA ~42

IMAGE RECONSTRUCTION UNIT ~44

10

CEREBRAL BLOOD FLOW RATE QUANTIFICATION DEVICE

12~ NUCLEAR MEDICINE IMAGE ACQUISITION UNIT

TIME INPUT UNIT ~14

16~ COUNT CALCULATION UNIT

RADIOACTIVITY COUNT INPUT UNIT ~20

18~ INPUT FUNCTION DETERMINATION UNIT

STANDARD INPUT FUNCTION STORAGE ~22

24~ CEREBRAL BLOOD FLOW RATE CALCULATION UNIT

26~ TABLE GENERATION UNIT

TABLE STORAGE ~28

30~ CEREBRAL BLOOD FLOW MAP GENERATION UNIT

【Fig.2】

```
                    ┌─────────┐
                    │  START  │
                    └────┬────┐
                         │
              ┌──────────▼──────────┐
              │   SPECT IMAGING     │～S10      ┐
              └──────────┬──────────┘           │
                         │                      │  OPERATION OF THE
              ┌──────────▼──────────┐           │  SPECT IMAGING
              │   GENERATION OF     │～S12      │  APPARATUS
              │ RECONSTRUCTION IMAGES│          ┘
              └──────────┬──────────┘
                         │
              ┌──────────▼──────────┐           ┐
              │   ACQUISITION OF    │～S14       │
              │ RECONSTRUCTION IMAGES│           │
              └──────────┬──────────┘           │
                         │                      │
              ┌──────────▼──────────┐           │
              │ INPUT OF START AND  │～S16       │
              │    FINISH TIME      │           │
              └──────────┬──────────┘           │
                         │                      │
              ┌──────────▼──────────┐           │
              │  CALCULATION OF THE │～S18       │
              │  NUMBER OF COUNTS IN │          │
              │    EACH PIXEL       │           │
              └──────────┬──────────┘           │  OPERATION OF THE
                         │                      │  CEREBRAL BLOOD FLOW
              ┌──────────▼──────────┐           │  QUANTIFICATION DEVICE
              │ DETERMINATION OF THE│～S20       │
              │  INPUT FUNCTION     │           │
              └──────────┬──────────┘           │
                         │                      │
              ┌──────────▼──────────┐           │
              │ GENERATION OF THE TABLE│～S22    │
              └──────────┬──────────┘           │
                         │◄──────┐              │
              ┌──────────▼──────────┐           │
              │ READING OF THE CEREBRAL│～S24    │
              │   BLOOD FLOW RATE   │           │
              └──────────┬──────────┘           │
                         │                      │
                    ◇────▼────◇                 │
              HAS THE READING                   │
              FINISHED FOR ALL ◇～S26            │
                   PIXELS?                       │
                    ◇────┬────◇                 │
                         │                      │
              ┌──────────▼──────────┐           │
              │  GENERATION OF THE  │～S28       │
              │ CEREBRAL BLOOD FLOW MAP│        ┘
              └──────────┬──────────┘
                         │
                    ┌────▼────┐
                    │   END   │
                    └─────────┘
```

14

【Fig.3】

50

PROGRAM FOR QUANTIFYING THE
CEREBRAL BLOOD FLOW RATE

NUCLEAR MEDICINE IMAGE
ACQUISITION MODULE ~52

TIME INPUT MODULE ~54

COUNT CALCULATION MODULE ~56

INPUT FUNCTION DETERMINATION MODULE ~58

RADIOACTIVITY COUNT INPUT MODULE ~60

STANDARD INPUT FUNCTION DATA ~62

CEREBRAL BLOOD FLOW RATE CALCULATION
MODULE ~64

TABLE GENERATION MODULE ~66

CEREBRAL BLOOD FLOW MAP GENERATION
MODULE ~68

【Fig.4】

**INTERNATIONAL SEARCH REPORT**

| International application No. |
| --- |
| PCT/JP2009/000121 |

A. CLASSIFICATION OF SUBJECT MATTER
*G01T1/161*(2006.01)i

According to International Patent Classification (IPC) or to both national classification and IPC

B. FIELDS SEARCHED

Minimum documentation searched (classification system followed by classification symbols)
G01T1/161

Documentation searched other than minimum documentation to the extent that such documents are included in the fields searched
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2006-308527 A (President of National Cardiovascular Center), 09 November, 2006 (09.11.06), Par. No. [0154] (Family: none) | 1-8 |
| A | JP 2007-113945 A (Joji NAKAGAWARA), 10 May, 2007 (10.05.07), (Family: none) | 1-8 |
| A | JP 8-15439 A (Shimadzu Corp.), 19 January, 1996 (19.01.96), (Family: none) | 1-8 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search 13 February, 2009 (13.02.09) | Date of mailing of the international search report 24 February, 2009 (24.02.09) |
| --- | --- |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2009/000121 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 11-76180 A  (Shimadzu Corp.),<br>23 March, 1999 (23.03.99),<br>(Family: none) | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008031922 A

**Non-patent literature cited in the description**

- **Hidehiro lida et al.** Quantitative mapping of regional cerebral blood flow using iodine-123-IMP and SPECT. *the Journal of Nuclear Medicine,* December 1994, vol. 35 (12), 2019-2030
- **Hidehiro lida et al.** A method to quantitate cerebral blood flow using a rotating gamma camera and iodine-123 iodoamphetamine with one blood sampling. *European Journal of Nuclear Medicine,* October 1994, vol. 21 (10), 1072-1084
- **Takenori Hachiya et al.** Stability of cross calibration between SPECT apparatuses and well counters (SPECT souchi to well counter no cross calibration no anteisei). *the Japanese Journal of Nuclear Medicine Technology,* 1995, vol. 15 (2), 105-109
- **Takenori Hachiya et al.** Source Error Factors in Cross Calibration for Quantitative Single Photon Emission Computed Tomography. *the Japanese Journal of Nuclear Medicine Technology,* 1996, vol. 16 (2), 58-62